# EUROPÄISCHE PATENTSCHRIFT

(11) **EP 2 995 323 B1**
(45) Veröffentlichungstag und Bekanntmachung des Hinweises auf die Patenterteilung: **27.02.2019**
(21) Anmeldenummer: 14184703.8
(22) Anmeldetag: 15.09.2014
(51) Int. Cl.: A61L 15/24, A61L 15/22, A61L 15/60, C08F 2/44, C08F 6/00, C08K 5/092, C08L 101/14, B01J 20/26, B01J 20/30, C08L 33/02

(54) **Aminopolycarboxylsäuren als Prozesshilfsmittel bei der Superabsorberherstellung**
Amino polycarboxylic acids as processing aids in the production of superabsorbents
Acides aminés polycarboxyliques comme agents auxilaires de traitement dans la préparation de substances superabsorbantes

(43) Veröffentlichungstag der Anmeldung: 16.03.2016
(73) Patentinhaber: Evonik Degussa GmbH, 45128 Essen (DE)
(72) Erfinder: Loick, Christoph, 47918 Tönisvorst (DE); Loy, Ingo, 47839 Krefeld (DE); Gartz, Dominik, 41751 Viersen (DE)

(56) Entgegenhaltungen:
- EP-A1- 2 116 571
- EP-A1- 2 163 302
- EP-A1- 2 208 756
- EP-A1- 2 371 869
- EP-A1- 2 727 953
- WO-A1-2010/057912

## Beschreibung

Die vorliegende Erfindung liegt auf dem Gebiet der wasserabsorbierenden Polymerpartikel und Superabsorber. Sie betrifft ein Verfahren zur Herstellung wasserabsorbierender Polymerpartikel unter Einsatz von bestimmten Aminopolycarboxylsäuren und/oder deren Salzen.

Superabsorber sind bekannt und ist die Bezeichnung für vernetzte hydrophile Polymere, die große Mengen wässriger Flüssigkeiten aufnehmen können. Diese Fähigkeit beruht auf der starken Wechselwirkung von Wasser mit hydrophilen Gruppen der Superabsorber, insbesondere ionischer Gruppen oder Gruppen, die zur Wasserstoff-Brückenbindung befähigt sind. Für Superabsorber sind u.a. auch Bezeichnungen wie "hochquellfähiges Polymer", "Hydrogel" (oft auch für die trockene Form verwendet), "Hydrogel bildendes Polymer", "Wasser absorbierendes Polymer", "absorbierendes gelbildendes Material", "quellfähiges Harz", "wasserabsorbierendes Harz" oder ähnliche gebräuchlich. Insbesondere handelt es sich um wasserabsorbierende Polymere auf Basis teilneutralisierter Acrylsäure. Die wesentlichen Eigenschaften von Superabsorbern sind ihre Fähigkeiten, ein Vielfaches ihres Eigengewichts an wässrigen Flüssigkeiten zu absorbieren (z. B. das 30-800-fache) und die Flüssigkeit auch unter gewissem Druck nicht wieder abzugeben. Der Superabsorber, der in Form eines trockenen Pulvers eingesetzt wird, wandelt sich bei Flüssigkeitsaufnahme in ein Gel, bei der üblichen Wasseraufnahme entsprechend in ein Hydrogel um. Die Vernetzung ist für synthetische Superabsorber wesentlich und führt zur Unlöslichkeit der Polymeren in Wasser. Lösliche Substanzen wären als Superabsorber nicht brauchbar. Das mit weitem Abstand wichtigste Einsatzgebiet von Superabsorbern ist das Absorbieren von Körperflüssigkeiten. Superabsorber werden beispielsweise in Windeln für Kleinkinder, Inkontinenzprodukten für Erwachsene oder Damenhygieneprodukten verwendet. Andere Anwendungsgebiete sind beispielsweise als Wasser zurückhaltende Mittel im landwirtschaftlichen Gartenbau, als Wasserspeicher zum Schutz vor Feuer, zur Flüssigkeitsabsorption in Lebensmittel-Verpackungen, als Kabelummantelungsmaterial für Tiefseekabel oder ganz allgemein zur Absorption von Feuchtigkeit.

Im Allgemeinen weist ein derartiger Superabsorber eine CRC ("Centrifuge Retention Capacity") von mindestens 5 g/g, vorzugsweise mindestens 10 g/g, in bevorzugter Form mindestens 20 g/g, insbesondere 30 g/g auf. Wichtig für einen Superabsorber ist nicht nur seine Absorptionskapazität, sondern auch die Fähigkeit, Flüssigkeit unter Druck zurückzuhalten (Retention, meist als "Absorption against Pressure" ("AAP") ausgedrückt) sowie die Permeabilität, also die Fähigkeit zur Flüssigkeitsweiterleitung im gequollenen Zustand. Gequollenes Gel kann die Flüssigkeitsweiterleitung zu noch nicht gequollenem Superabsorber behindern ("gel blocking"). Gute Weiterleitungseigenschaften für Flüssigkeiten besitzen beispielsweise Hydrogele, die im gequollenen Zustand eine hohe Gelfestigkeit aufweisen. Gele mit nur geringer Gelfestigkeit sind unter einem angewendetem Druck (Körperdruck) deformierbar, verstopfen Poren in einem Superabsorber / Cellulosefaser-Saugkörper und verhindern dadurch die Flüssigkeitsweiterleitung zu noch nicht oder nicht vollständig gequollenem Superabsorber und die Flüssigkeitsaufnahme durch diesen noch nicht oder nicht vollständig gequollenen Superabsorber. Eine erhöhte Gelfestigkeit wird in aller Regel durch einen höheren Vernetzungsgrad erreicht, wodurch allerdings die Absorptionskapazität des Produktes verringert wird. Eine effektive Methode zur Erhöhung der Gelfestigkeit stellt die Erhöhung des Vernetzungsgrads an der Oberfläche der Superabsorberpartikel gegenüber dem Inneren der Partikel dar. Dazu werden meist in einem Oberflächennachvernetzungsschritt getrocknete Superabsorberpartikel mit durchschnittlicher Vernetzungsdichte einer zusätzlichen Vernetzung in einer dünnen Oberflächenschicht ihrer Partikel unterworfen. Durch die Oberflächennachvernetzung steigt die Vernetzungsdichte in der Schale der Superabsorberpartikel, wodurch die Absorption unter Druckbelastung auf ein höheres Niveau gehoben wird. Während die Absorptionskapazität in der Oberflächenschicht der Superabsorberpartikel sinkt, weist ihr Kern durch das Vorhandensein beweglicher Polymerketten eine im Vergleich zur Schale verbesserte Absorptionskapazität auf, so dass durch den Schalenaufbau eine verbesserte Permeabilität gewährleistet wird, ohne dass gel blocking auftritt. Es ist ebenfalls bekannt, insgesamt höher vernetzte Superabsorber zu erzeugen und den Vernetzungsgrad im Inneren der Partikel gegenüber einer äußeren Schale der Partikel nachträglich zu verringern.

Die Herstellung von derartigen Superabsorbern (auch Superabsorber-Polymere genannt) basiert im Wesentlichen auf der Polymerisation von ethylenisch ungesättigten, säuregruppentragenden Monomeren, die wahlweise zumindest teilweise als Salz vorliegen, insbesondere auf der radikalischen Polymerisation von teilneutralisierter Acrylsäure, üblicherweise in Gegenwart von Vernetzern. Die radikalische Polymerisationsreaktion ist eine schnell ablaufende Reaktion und ein stark exothermer Prozess.

Während dieser Reaktion wird ein dreidimensionales Polymernetzwerk aufgebaut, welches je nach Prozessbedingungen und Reaktionsführung unterschiedliche makroskopische Eigenschaften aufweisen kann. Findet die Polymerisationsreaktion beispielsweise unter sehr starker Wärmeentwicklung in sehr kurzer Zeit statt, so kann es z.B. infolge von Kettenübertragungsreaktionen zu sogenannten Fehlstellen im dreidimensionalen Polymernetzwerk kommen, wodurch einige Superabsorber-Eigenschaften negativ beeinflusst werden. Beispielsweise kann es zu einer Erhöhung der nicht vernetzten, sogenannten löslichen Anteile kommen.

Findet die Reaktion hingegen zu langsam statt, z.B. durch eine fehlerhafte Initiierung oder durch falsche Temperaturführung, so können z.B. die sogenannten Restmonomer-Anteile als Folge eines mangelhaften Umsatzes deutlich ansteigen.

Grundsätzlich gibt es unterschiedliche Möglichkeit zur Steuerung der Polymerisationskinetik. So kann beispielsweise über die Zusammensetzung und Konzentration der Monomere, Initiatoren und Vernetzer Einfluss auf die Kinetik genommen werden. Infolge des großen Einflusses der Polymerisationskinetik auf die Produktqualität, besteht ein kontinuierlicher Bedarf an Steuerungsmöglichkeiten der Polymerisationskinetik.

Es ist bekannt, dass Rezepturen für Superabsorber Chelatbildner enthalten:
So offenbart EP 2116571 A1 den Chelatbildner ethylenediaminedisuccinic acid, also Ethylendiamin-dibernsteinsäure (EDDS). Zweck der Zugabe des Chelatbildners ist die Verbesserung der Farbstabilität des Superabsorbers. Die Zugabe erfolgt zu der Monomerlösung, also vor der Polymerisation. Die Reaktion wird wahlweise in einen Kneter oder auf einem Bandreaktor durchgeführt.

In der EP 2208756 A1 ist EDDS als Chelatbildner ebenfalls erwähnt. Als Polymerisationsmethoden gibt diese Schrift wahlweise Band- oder Knetreaktor-Polymerisation an. Production example 1 der EP 2208756 A1 wurde in einem Knetreaktor unter Zugabe von trisodium diethylenetriamine pentaacetate durchgeführt; Es wird damit ein AAP-Wert von 21 g/g erzielt. Die konkrete Aufgabe der vorliegenden Erfindung war es vor diesem Hintergrund, bei der Herstellung von wasserabsorbierenden Polymerpartikeln, umfassend die Polymerisation ethylenisch ungesättigter, säuregruppentragender Monomere, die wahlweise zumindest teilweise als Salz vorliegen, die Steuerung der Polymerisationskinetik zu ermöglichen.

Überraschenderweise konnte nun in Aminopolycarboxylsäuren und/oder deren Salzen, insbesondere in Ethylendiamindibernsteinsäure und/oder deren Salz, ein besonders wirksames Prozesshilfsmittel gefunden werden, das bei der Herstellung wasserabsorbierender Polymerpartikel, umfassend die Polymerisation ethylenisch ungesättigter, säuregruppentragender Monomer, die wahlweise zumindest teilweise als Salz vorliegen, insbesondere umfassend die Polymerisation von Acrylsäure, die zumindest teilweise als Natriumacrylat vorliegt, eine Steuerung der Polymerisationskinetik ermöglicht und so eine effektive Einflussnahme auf die Eigenschaften der wasserabsorbierenden Polymerpartikel gestattet. Damit wird eine besonders wirkungsvolle Möglichkeit zur Einstellung optimierter Eigenschaften der wasserabsorbierenden Polymerpartikel zur Verfügung gestellt.

Gegenstand der vorliegenden Erfindung, welcher die erfindungsgemäße Aufgabe löst, ist daher ein Verfahren zur Herstellung wasserabsorbierender Polymerpartikel mit den Merkmalen des Anspruch 1.

Bei Einsatz der Aminopolycarboxylsäuren, insbesondere Ethylendiamindibernsteinsäure, Ethylendiamintetraessigsäure, Diethylentriamintetraessigsäure, Glutaminsäure-N, N-diessigsäure, Methylglycindiessigsäure und/oder deren Salzen, wobei Ethylendiamindibernsteinsäure und/oder ein Salz davon bevorzugt sind, kann eine Inhibierung der Polymerisationsreaktion erreicht werden, wodurch eine Optimierung der Polymer-Netzwerkbildung möglich wird, so dass verbesserte Eigenschaften insbesondere bezüglich löslicher Anteile, CRC und AAP-Verhältnis erreicht werden können. Das Ausmaß der Inhibierung kann dabei über die Einsatzmenge an Aminopolycarboxylsäure und/oder deren Salz, vorzugsweise Ethylendiamindibernsteinsäure und/oder einem Salz davon, auf einfachem Wege eingestellt werden, so dass eine Steuerung der Polymerisationskinetik einfach möglich ist. Dazu sind für den Fachmann nur wenige orientierende Versuche notwendig.

Desweiteren ermöglicht die vorliegende Erfindung eine Verbesserung der Gelverarbeitung, insbesondere mit Blick auf die Gelzerkleinerung, den Gel-Transport sowie die Gel-Trocknung, da die Klebrigkeit des Gels reduziert und insbesondere minimiert wird.

Aminopolycarboxylsäuren und/oder deren Salze sind dem Fachmann an sich bekannt und kommerziell problemlos verfügbar. Das Salz der Aminopolycarboxylsäure ist vorzugsweise ein Alkalimetallsalz, Erdalkalimetallsalz, Ammoniumsalz, substituiertes Ammoniumsalz und/oder deren Gemisch, vorzugsweise ist es ein Natriumsalz.

Der Einsatz von Ethylendiamindibernsteinsäure und/oder einem Salz davon ist ein Merkmal der vorliegenden Erfindung.

Ethylendiamindibernsteinsäure (Ethylendiamindisuccinat) besitzt zwei Stereozentren, so dass es in drei verschiedenen Stereoisomeren auftreten kann, als (S,S)-, (R,R)- und als meso-EDDS. Ein einfacher, großindustriell leicht gangbarer Zugang zu EDDS stellt z.B. die Addition von Ethylendiamin an Maleinsäureanhydrid dar. Auf diesem Weg erhält man ein Stereoisomerengemisch, welches aus den (S,S)- und dem (R,R)-Isomeren sowie außerdem dem (R,S)-Isomer (= meso-Form) besteht. Eine gezielte Synthese für (S,S)-EDDS besteht z.B. in der Umsetzung von L-Asparaginsäure mit 1,2-Dibromethan. Für die Zwecke der vorliegenden Erfindung sind alle drei Stereoisomeren jeweils einzeln sowie jegliche Stereoisomerengemische einsetzbar. Der Einsatz von (S,S)-EDDS ist bevorzugt.

Erfindungsgemäß wird Ethylendiamindibernsteinsäure und/oder ein Salz davon eingesetzt. Grundsätzlich können 1 bis 4 der Carboxylgruppen der Ethylendiamindibernsteinsäure in Salzform vorliegen. So sind beispielsweise Mono-, Di-, Tri- oder Tetrasalze herstellbar. In den Salzen liegt als Gegenion das Ion der entsprechenden verwendeten Base vor. So können z.B. bei Verwendung von Natriumcarbonat oder Natriumhydroxid Mono-, Di-, Tri- und Tetranatriumsalze von EDDS erhalten werden. Bei Verwendung der entsprechenden Kaliumverbindungen werden die entsprechenden Kaliumsalze erhalten, usw. In Produktgemischen können auch im Mittel ungeradzahlige Werte für die Anzahl an Salzgruppen im Molekül erhalten werden.

Das Salz der Ethylendiamindibernsteinsäure ist vorzugsweise ein Alkalimetallsalz, Erdalkalimetallsalz, Ammoniumsalz, substituiertes Ammoniumsalz und/oder deren Gemisch, vorzugsweise ist es ein Natriumsalz, insbesondere das Tetranatriumsalz. "EDDS" bedeutet im Sinne dieser Erfindung Ethylendiamindibernsteinsäure und/oder ein Salz davon.

Auch Ethylendiamintetraessigsäure, Diethylentriamintetraessigsäure, Glutaminsäure-N, N-diessigsäure, Methylglycindiessigsäure und/oder deren Salze sind dem Fachmann gut bekannt und bedürfen daher keiner weiteren Erläuterung. Insbesondere die betreffenden Alkalimetallsalze können mit Vorteil eingesetzt werden.

Insbesondere ermöglicht der erfindungsgemäße Einsatz der Aminopolycarboxylsäuren und/oder deren Salze, insbesondere von Ethylendiamindibernsteinsäure und/oder einem Salz davon, die Beeinflussung der Wärmeentwicklung bei der Polymerisation und zwar vorzugsweise die Reduktion der Wärmeentwicklung in der Startphase nach Initiierung der Polymerisation, vorzugsweise in den ersten 600 Sekunden nach Start der Polymerisation. Vorteilhafterweise ermöglicht der erfindungsgemäße Einsatz der Aminopolycarboxylsäuren und/oder deren Salze, insbesondere von Ethylendiamindibernsteinsäure und/oder einem Salz davon, die Reduktion der nicht vernetzten löslichen Anteile im Polymerisationsendprodukt, die Verbesserung des CRC/AAP-Verhältnisses im Polymerisationsendprodukt sowie die Verbesserung der Gelfließfähigkeit des ungetrockneten Polymerisates.

Der Einsatz von Ethylendiamindibernsteinsäure und/oder einem Salz davon führt jeweils zu ganz besonders vorteilhaften Ergebnissen im Sinne dieser Erfindung.

Die Zugabe des erfindungsgemäßen Chelatbildners, also von Ethylendiamindibernsteinsäure und/oder deren Salz, kann vor und/oder während der Polymerisation erfolgen. Insbesondere kann die Ethylendiamindibernsteinsäure und/oder deren Salz, der zu polymerisierenden Monomerlösung oder -suspension vor dem Start der Polymerisation zugegeben werden.

Der erfindungsgemäße Chelatbildner, also Ethylendiamindibernsteinsäure und/oder deren Salz, kann in fester, partikulärer Form und/oder in Form einer vorzugsweise wässrigen Lösung der Monomerlösung oder -suspension zugesetzt werden, was einer bevorzugten Ausführungsform der Erfindung entspricht. Dabei ist die Zugabe in Form einer wässrigen Lösung bevorzugt.

Die Einsatzmengen an erfindungsgemäßen Chelatbildner, also Ethylendiamindibernsteinsäure und/oder deren Salz, können je nach den individuellen Bedürfnissen der jeweils gegebenen Prozessbedingungen und Reaktionsverhältnisse gewählt werden, wobei der Fachmann mit Hilfe weniger orientierender Versuche die gewünschte Reaktionskinetik ganz nach Bedarf einstellen kann.

Wenn erfindungsgemäße Chelatbildner, also Ethylendiamindibernsteinsäure und/oder deren Salz, im Rahmen dieser Erfindung in einer Gesamtmenge von zumindest 5 ppm, vorzugsweise 50 bis 2000 ppm, insbesondere 100 bis 1500 ppm, bezogen auf die Menge an unneutralisiertem Monomer der Monomerlösung oder -suspension zugesetzt werden, so liegt eine bevorzugte Ausführungsform der Erfindung vor. Mit den vorgenannten Mengen ist eine besonders wirkungsvolle Steuerung der Reaktionskinetik möglich und insbesondere kann eine gewünschte zeitliche Verlangsamung der Polymerisationsstartphase erzielt werden.

Die vorliegende Erfindung erlaubt die Steuerung der Polymerisationskinetik bei der Herstellung von Superabsorber-Polymeren, umfassend die Polymerisation ethylenisch ungesättigter, säuregruppentragender Monomer, die zumindest teilweise als Salz vorliegen.

Die Wirkmächtigkeit des erfindungsgemäßen Prozesshilfsmittels hat sich allerdings in besonders eindrucksvollem Maße bei der Polymerisation von Acrylsäure bewährt, die zumindest teilweise als Natriumacrylat vorliegt.

Wenn in dem erfindungsgemäßen Verfahren das Monomer a) Acrylsäure ist, die zumindest teilweise als Natriumacrylat vorliegt, so liegt also eine bevorzugte Ausführungsform der Erfindung vor. Zu den einsetzbaren Monomeren werden weiter unten noch genauere Ausführungen gemacht.

Grundsätzlich können im Rahmen der vorliegenden Erfindung die üblichen Vernetzer und Vernetzermengen bei der Polymerisation zum Einsatz gelangen. Hierzu werden weiter unten noch genauere Ausführungen gemacht. Wenn die Monomerlösung oder -suspension mindestens 0,1 Gew.-% Vernetzer b), bezogen auf unneutralisiertes Monomer a) enthält, so liegt allerdings eine bevorzugte Ausführungsform der Erfindung vor.

Weiterhin entspricht es einer bevorzugten Ausführungsform der Erfindung, wenn im Rahmen des erfindungsgemäßen Verfahrens eine Oberflächennachvernetzung durchgeführt wird. Auch dies wird weiter unten noch genauer erläutert.

Weiterhin ist es bevorzugt, dass man das vorzugsweise oberflächennachvernetze Polymerisat einer weiteren Behandlung unterzieht, vorzugsweise Oberflächenbehandlung, insbesondere durch Zugabe mindestens eines Nachbehandlungsmittels. Dies entspricht einer Nachbehandlung des ggf. oberflächennachvernetzten Polymerisats, vorzugsweise mit zumindest einem Nachbehandlungsmittel. Geeignete Nachbehandlungsmittel sind insbesondere Stoffe, die dem Polymerisat zugesetzt werden, um dessen Eigenschaften in gewünschter Richtung zu verändern und/oder dessen Verarbeitbarkeit zu erleichtern. Geeignete Nachbehandlungsmittel sind dem Fachmann aus der Superabsorbertechnologie wohlbekannt. Es handelt sich z.B. um Anti-Staub-Mittel, wie beispielsweise Polyole und/oder oder Polyalkylenglykole, um Anti-Backmittel, wie beispielsweise Sipernate, Aerosile kationische Tenside, so z.B. quatemäre Ammonium- oder Phosphonium-Salze, um Geruchskontroll-Mittel, wie z.B. Zeolithe, Bentonite, Silica, Cyclodextrine, Riechstoffe, antimikrobielle Wirkstoffe oder Oxidationsmittel.

Obschon die Herstellung wasserabsorbierender Polymerpartikel an sich wohlbekannt ist, wird im Folgenden das Herstellungsverfahren noch näher erläutert, denn die nachfolgend beschriebenen bevorzugten Ausführungsformen führen zu besonders guten Ergebnissen mit Blick auf die Lösung der angestrebten Aufgabe.

Das erfindungsgemäße Verfahren umfasst, wie schon ausgeführt, die Polymerisation einer Monomerlösung oder -suspension, die
a) mindestens ein ethylenisch ungesättigtes, säuregruppentragendes Monomer, das wahlweise zumindest teilweise als Salz vorliegt,
b) mindestens einen Vernetzer,
c) mindestens einen Initiator,
d) wahlweise ein oder mehrere mit den unter a) genannten Monomeren copolymerisierbare ethylenisch ungesättigte Monomere,
e) wahlweise ein oder mehrere wasserlösliche Polymere
f) Wasser
g) wahlweise Additive und/oder Wirksubstanzen
enthält.

Die dabei einsetzbaren Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete einsetzbare Monomere a) sind beispielsweise ethylenisch ungesättigte Carbonsäuren, wie Acrylsäure, Methacrylsäure, und Itaconsäure. Besonders bevorzugte einsetzbare Monomere sind Acrylsäure und Methacrylsäure. Ganz besonders bevorzugt ist Acrylsäure.

Weitere geeignete Monomere a) sind beispielsweise ethylenisch ungesättigte Sulfon- säuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes einsetzbares Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfural, 0,0001 Gew.-% Maleinsäureanhydrid, 0,0003 Gew.-% Diacrylsäure und 0,0050 Gew.-% Hydrochinonmonomethylether. Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%. Die Monomere a) können üblicherweise Polymerisationsinhibitoren, vorzugsweise Hydrochinonhalbether, als Lagerstabilisator enthalten.

Die einsetzbare Monomerlösung kann vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether enthalten, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).
Geeignete einsetzbare Vernetzer b) sind z.B. Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind z.B. auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldi- acrylat, Polyethylenglykoldiacrylat, Allylmethacrylat, Trimethylolpropantriacrylat, Trially- lamin, Tetraallylammoniumchlorid, Tetraallyloxyethan, wie in EP 0 530 438 A1 beschrieben, Di- und Triacrylate, wie in EP 0 547 847 A1 , EP 0 559 476 A1 , EP 0 632 068 A1 , WO 93/21237 A1 , WO 2003/104299 A1 , WO 2003/104300 A1 , WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1 , DE 196 46 484 A1 , WO 90/15830 A1 und WO 2002/032962 A2 beschrieben.

Bevorzugte Vernetzer b) sind Pentaerythrittriallylether, Tetraalloxyethan, Methylenbismethacrylamid, 15-fach ethoxiliertes Trimethylolpropantriacrylat, Polyethylenglykoldiacrylat, Trimethylolpropantriacrylat und Triallylamin. Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind.

Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1 ,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck durchläuft ein Maximum.

Als einsetzbare Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind z.B. Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; DE) erhältlich.

Mit den ethylenisch ungesättigten, säuregruppentragenden Monomeren a) copolymerisierbare ethylenisch ungesättigte Monomere d) sind beispielsweise Acrylamid, Methacrylamid Hydroxyethylacrylat, Hydroxyethylmethacrylat, Dimethylaminoethylmethacrylat Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können z.B. Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise kann eine wässrige Monomerlösung verwendet werden. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugt einsetzbaren Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete, in der Monomerlösung oder -suspension optional enthaltene Additive und/oder Wirksubstanzen, sind Stoffe, welche die Eigenschaften der resultierenden Polymerisate in gewünschter Richtung verändern können, deren Verarbeitbarkeit erleichtern können oder das Polymerisat mit einer Zusatzwirkung ausstatten können. Hierbei kann auf alle aus dem Stand der Technik gebräuchlichen Additive und/oder Wirksubstanzen zurückgegriffen werden.

Geeignete Reaktoren sind Knetreaktoren. Im Kneter wird das bei der Polymerisation einer wässrigen Monomerlösung oder -Suspension entstehende Polymergel durch beispielsweise gegenläufige Rührwellen kontinuierlich zerkleinert, wie z.B. in WO 2001/038402 A1 beschrieben.Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 85 mol-%, für "saure" Polymergele besonders bevorzugt von 30 bis 60 mol-%, ganz besonders bevorzugt von 35 bis 55 mol-%, für "neutrale" Polymergele besonders bevorzugt von 65 bis 80 mol-%, ganz besonders bevorzugt von 70 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze wie das Salz des Triethanolamins verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden. Das Polymergel kann dann vorzugsweise mit einem Bandtrockner getrocknet werden bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA (European Disposables and Nonwovens Association) empfohlenen Testmethode Nr. WSP 230.2-05 "Moisture Content" bestimmt wird. Die EDANA-Testmethoden sind beispielsweise erhältlich bei der EDANA, Avenue Eugene Plasky 157, B-1030 Brüssel, Belgien.

Bei einer zu hohen Restfeuchte kann das getrocknete Polymergel eine zu niedrige Glasübergangstemperatur TG aufweisen und ist dann nur schwierig weiter zu verarbeiten. Bei einer zu niedrigen Restfeuchte kann das getrocknete Polymergel zu spröde sein und in den anschließenden Zerkleinerungsschritten können unerwünscht große Mengen an Polymerpartikeln mit zu niedriger Partikelgröße ("fines") anfallen. Der Feststoffgehalt des Gels beträgt vor der Trocknung vorzugsweise von 25 bis 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach vorzugsweise gemahlen und klassiert, wobei zur Mahlung üblicherweise ein- oder mehrstufige Walzenstühle, bevorzugt zwei- oder dreistufige Walzenstühle, Schneidmühle, Ultra-Zentrifugalmühle Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 µm, besonders bevorzugt von 250 bis 600 µm, ganz besonders von 300 bis 500 µm. Die mittlere Partikelgröße der Produktfraktion kann mittels der von der EDANA (European Disposables and Nonwovens Association) empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 µm beträgt vorzugsweise mindestens 90 Gew.-%, besonders bevorzugt mindestens 95 Gew.-%, ganz besonders bevorzugt mindestens 98 Gew.-%.

Polymerpartikel mit zu niedriger Partikelgröße senken die Permeabilität. Daher sollte der Anteil zu kleiner Polymerpartikel ("fines") niedrig sein. Zu kleine Polymerpartikel werden daher üblicherweise abgetrennt und in das Verfahren rückgeführt. Die geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet (was der Erfindungsgemäßen Lehre entspricht), so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 µm beträgt vorzugsweise mindestens 90 Gew.-%, besonders bevorzugt mindestens 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein. Zu große Polymerpartikel werden daher üblicherweise abgetrennt und in die Mahlung des getrockneten Polymergels rückgeführt. Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1 , DE 35 23 617 A1 und EP 0 450 922 A2 beschrieben, oder ß-Hydroxyalkylamide, wie in DE 102 04 938 A1 und US 6,239,230 beschrieben. Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 Ci Bis- und Poly-2-oxazolidinone, in DE 198 54 573 A1 2-Oxotetrahydro-1 ,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben. Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1 ,4-Butandiol. Ganz besonders bevorzugte Oberflächennachvernetzer sind 2-Hydroxyethyloxazolidin- 2-on, Oxazolidin-2-on und 1 ,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben.

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,75 Gew.-%, jeweils bezogen auf die Polymerpartikel.

In einer bevorzugten Ausführungsform der vorliegenden Erfindung werden vor, während und/oder nach der Oberflächennachvernetzung zusätzlich zu den Oberflächennachvernetzern polyvalente Kationen auf die Partikeloberfläche aufgebracht. Die einsetzbaren polyvalenten Kationen sind beispielsweise zweiwertige Kationen, wie die Kationen von Zink, Magnesium, Kalzium und Strontium, dreiwertige Kationen, wie die Kationen von Aluminium, vierwertige Kationen, wie die Kationen von Titan und Zirkonium. Als Gegenion sind beispielsweise Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat und Aluminiumlaktat sind bevorzugt. Außer Metallsalzen können auch Polyamine als polyvalente Kationen eingesetzt werden. Die Einsatzmenge an polyvalentem Kation beträgt beispielsweise 0,001 bis 1 ,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel augegeben, vorzugsweise aufgesprüht wird. Im Anschluss daran werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Schugi Flexomix® (Hosokawa Micron BV; Doetinchem; NL). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1 ,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; DE), Hosokawa Bepex® Disc Dryer (Hosokawa Micron GmbH; Leingarten; DE) und Nara Paddle Dryer (NARA Machinery Europe; Frechen; DE). Überdies können auch Wirbelschichttrockner eingesetzt werden. Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C bevorzugt 120 bis 220 °C besonders bevorzugt 130 bis 210 °C ganz besonders bevorzugt 150 bis 200 °C Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die vorzugsweise oberflächennachvernetzten Polymerpartikel können zur weiteren Verbesserung der Eigenschaften nachbehandelt werden, vorzugsweise mit einem Nachbehandlungsmittel nachbehandelt werden, insbesondere beschichtet und/oder nachbefeuchtet werden.

Die optionale Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur optionalen Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die optionale Nachbefeuchtung wird die mechanische Stabilität der Polymerpartikel erhöht und deren Neigung zur statischen Aufladung vermindert. Geeignete optionale Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete optionale Beschichtungen zur Staubbindung sind beispielsweise Polyole. Geeignete optionale Beschichtungen gegen die unerwünschte Verbackungsneigung der Polymerpartikel sind beispielsweise pyrogene Kieselsäure, wie Aerosil® 200, und Tenside, wie Span® 20.

Die wasserabsorbierenden Polymerpartikel weisen einen Feuchtegehalt von vorzugsweise 1 bis 15 Gew.-%, besonders bevorzugt 2 bis 10 Gew.-%, ganz besonders bevorzugt 3 bis 5 Gew.-%, auf, wobei der Feuchtegehalt gemäß der von der EDANA (European Disposables and Nonwovens Association) empfohlenen Testmethode Nr. WSP 230.2-05 "Moisture Content" bestimmt wird.

Die im Rahmen dieser Erfindung vorteilhafterweise resultierenden wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der im Rahmen dieser Erfindung vorteilhafterweise resultierenden wasserabsorbierenden Polymerpartikel beträgt üblicherweise weniger als 60 g/g. Die Zentrifugenretentionskapazität (CRC) wird gemäß der von der EDANA (European Disposables and Nonwovens Association) empfohlenen Testmethode Nr. WSP 241.3-10 "Centrifuge retention capacity" bestimmt. Die im Rahmen dieser Erfindung resultierenden wasserabsorbierenden Polymerpartikel weisen eine Absorption unter einem Druck von 49,2 g/cm2 (0,7 psi) von mindestens 22 g/g, besonders bevorzugt mindestens 23,5 g/g, ganz besonders bevorzugt mindestens 25 g/g, auf. Die Absorption unter einem Druck von 49,2 g/cm2 (0,7 psi) der wasserabsorbierenden Polymerpartikel beträgt üblicherweise weniger als 35 g/g. Die Absorption unter einem Druck (Absorption Against Pressure, AAP), hier unter einem Druck von 49,2 g/cm2 (0,7 psi), wird gemäß der Edana-Methode Nr. WSP242.3-10 bestimmt.

Im Rahmen der vorliegenden Erfindung wird die Polymerisation in einem Kneter durchgeführt, wobei die Zugabe des EDDS zu der Monomerlösung oder -suspension vor der Polymerisation erfolgt und/oder wobei die Zugabe des EDDS während der Polymerisation zu dem Inhalt des Kneters erfolgt.

Dabei ist es erfindungsgemäß, dass der Kneter mit wenigstens zwei parallelen Wellen ausgestattet ist und Elemente an wenigstens einer Welle aufweist, die den Inhalt des Kneters parallel zu den Wellen von einem Zufuhrabschnitt zu einem Ausstoßabschnitt transportieren.

Weiterhin kann der Kneter im Rahmen einer bevorzugten Ausführungsform der Erfindung im Batch-Betrieb benutzt werden.

Weiterhin hat es sich als vorteilhaft erweisen, wenn gemäß einer bevorzugten Ausführungsform der Erfindung der Gehalt der Monomerlösung oder -suspension an Eisen-Ionen unter 5 ppm, vorzugsweise unter 3 ppm und in besonders bevorzugter Weise unter 1 ppm liegt.

Die vorliegende Erfindung ermöglicht die Bereitstellung von Artikeln, welche wasserabsorbierende Polymerpartikel enthalten.
Demgemäße Artikel sind z.B. jegliche Inkontinenzhilfsmittel, wie insbesondere Windeln sowie Hygieneartikel, wie insbesondere Damenbinden und Tampons.

### Beispiele:

### Testmethoden:

Alle Testmethoden werden im Rahmen dieser Erfindung grundsätzlich, wenn nichts anderes angegeben ist, bei einer Umgebungstemperatur von 23+2 °C und einer relativen Luftfeuchte von 50+10 % durchgeführt. Die wasserabsorbierenden Polymerpartikel werden vor der Messung gut durchmischt.

Zentrifugenretentionskapazität (CRC Centrifuge Retention Capacity) :
Die Bestimmung der Zentrifugenretentionskapazität erfolgte gemäß der EDANA (European Disposables and Nonwovens Association) empfohlenen Testmethode Nr. WSP 241.3-10 "Centrifuge retention capacity".

Absorption gegen einen Druck von 0,7 psi (AAP):
Das Flüssigkeitsaufnahmevermögen gegen einen äußeren Druck (Absorption Against Pressure, AAP), hier von 0,7 psi, wurde gemäß der Edana-Methode Nr. WSP242.3-10 bestimmt.

Restmonomerengehalt:
Der Gehalt der Superabsorberpartikel an Restmonomeren wurde gemäß der EDANA Standard-Testmethode Nr. WSP210.3-10 bestimmt.

Lösliche Anteile, 16 h - Wert:
Der Anteil der Superabsorberpartikel an löslichen Anteilen nach 16 Stunden wurde gemäß der EDANA Standard-Testmethode Nr. WSP270.3-10 bestimmt.

Bestimmung des max. Differenzdruckes an den Kneterwellen und der maximalen Temperatur während der Polymerisation:
Die Temperatur des Polymerisats wurde während der gesamten Verweildauer des Polymers im Reaktor mittels eines in den Reaktionsraum hereinragenden Thermofühler ermittelt. Dabei wurde der während der Polymerisationsreaktion auftretende maximale Wert ermittelt. Der Differenzdruck an den Wellen des Kneterreaktors wurde über einen Druckaufnehmer ermittelt und ist ein Maß für das Drehmoment und die Scherkräfte im Reaktionsraum. Bei erhöhtem Differenzdruck sind die Drehmomente und somit Scherkräfte im Reaktionsraum vergrößert. Der maximale Wert, der während der Verweilzeit des Polymerisats im Kneterreaktor auftrat, wurde ermittelt.

### Herstellvorschrift:

### a) Precursor-Polyacrylat

Es wurde eine Monomerlösung bestehend aus 2400,00 g Acrylsäure, 2853,37 g 32%iger Natronlauge Lösung, 1510,82g vollentsalztem Wasser, 15,99 g 60%iger Polyethylenglykol(600) Lösung und eine wässrige Lösung eines Chelatbildners durch Spülen mit Stickstoff vom gelösten Sauerstoff befreit. Die Stickstoffspülung wurde ca. 10 Minuten lang durchgeführt. Kurz vor Überführung der Monomerlösung in den Knetreaktor, wurden der Monomerlösung unter weiterer Inertgasspülung zunächst 23,99 g Natriumsulfat und 197,84 g teilneutralisierter oberflächennachvernetzte Polyacrylsäure mit der durchschnittlichen Partikelgröße von < 150 µm (entspricht dem Feinanteil typischer Polyacrylatpartikel) zugeführt. Unter weiterem Rühren erfolgte die Zugabe von 6,95 g 69%iger Monoallyletherpolyethylenglykol-450-monoacrylsäureester Lösung sowie 2,40 g 15[EO]-Trimethylolpropantriacrylat.

Dann erfolgte der Transfer der Monomerlösung im Inertgasgegenstrom in den, durch Mantel- und Wellenheizung auf 35°C temperierten Polymersationsreaktor. Der Polymerisationsreaktor bestand aus einem zweiwelligen, gleichläufig drehenden, diskontinuierlich betriebenen KneterReaktor der Firma LIST AG (CH-4422 Arisdorf, Schweiz). Während des Transports der Monomerlösung durch einen leichten Unterdruck im System, betrug die Wellengeschwindigkeit 60Upm, sowie beim Einzug von 359,82 g 10%iger Natriumcarbonat Lösung. Darauf wurde das System druckentlastet, mit Inertgas überlagert und die Zugabe von 35,98 g einer 10 %igen wässrigen Natriumperoxodisulfat Lösung und 17,99 g einer 6 %igen wässrigen Wasserstoffperoxid-Lösung erfolgte.

Die Polymerisation wurde durch die Zugabe von 85,67 g einer 0,70 %igen wässrigen Ascorbinsäurelösung gestartet. Direkt nach dem Start der Reaktion wurde die Mantel- und Wellen-Beheizung auf den Sollwert von 75°C geregelt. Die Wellen des Reaktors wurden auf 25 Umdrehungen pro Minute gedrosselt. Eine exotherme Polymerisationsreaktion fand statt und die Inergasspühlung wurde beendet. Die Verweilzeit des Reaktionsgemisches betrug 20 Minuten. Kurz vor der Entleerung des Reaktors wurde die Mantel- und Wellentemperatur auf einen Sollwert von 35°C geregelt. Ohne weitere Zerkleinerungsschritte wurde das entstandene Hydrogel im Labor-Umlufttrockenschrank portionsweise 30 Minuten bei 190 °C getrocknet. Anschließend wurde das getrocknete Polymerisat in einer Schneidmühle (2mm) zerkleinert und die Partikelgrößen des zerkleinerten, getrockneten Superabsorbers von 150 µm bis 850 µm durch Siebung gewonnen.

### b) Oberflächen-Nachvernetzung:

Eine anschließende Oberflächen-Nachvernetzung wurde mit getrockneten, gemahlenen und abgesiebten Polymerpartikeln (= Precursormaterial) aus der oben beschrieben Herstellvorschrift vollzogen.

Hierzu wurde die Oberflächen-Nachvernetzungslösung, bestehend aus Ethylencarbonat/Wasser (1 bzw. 3 Gewichtsprozent bezogen auf Superabsorbermasse) auf das Precursormaterial mittels einer Einwegspritze aufgesprüht und im Krupps-Mixer ca. 1 Minute vermengt. Anschließend erfolgte dann eine Trocknung/Oberflächennachvernetzung im Labortrockenschrank bei 180°C/30 Minuten. Eine erneute Klassierung der Fraktionen erfolgte, sowie eine Homogenisation der generierten Superabsorberprobe.

Die nachfolgenden Beispiele wurden unter Rückgriff auf die vorgenannte Herstellvorschrift ausgeführt:
Referenz (Ansatz ohne Zusatz von Chelatbildnern) - nicht erfindungsgemäß
Es wurde keine wässrige Chelatbildner-Lösung zugesetzt.

### Beispiel 1: (Ansatz mit DTPA-Pentanatriumsalz als Prozesshilfsmittel) - nicht erfindungsgemäß

Als Chelatbildner wurden 1,19 g einer 40,2Gew%igen wässrigen Lösung aus Diethylentriaminpentaacetat-Natriumsalz (Versenex® 80E ex Dow) zu der Monomerlösung gegeben.

### Beispiel 2a (Ansatz mit EDTA-Tetranatriumsalz = Trilon® B liquid ex BASF als Prozesshilfsmittel) - nicht erfindungsgemäß

Als Chelatbildner wurden 0,85 g einer 42,5Gew%igen wässrigen Lösung aus Ethylendiamintetraacetat-Natriumsalz (Trilon® B liquid ex BASF) zu der Monomerlösung gegeben.

### Beispiel 2b (Ansatz mit EDTA-Tetranatriumsalz = Trilon® B liquid ex BASF als Prozesshilfsmittel) - nicht erfindungsgemäß

Als Chelatbildner wurden 1,70 g einer 42,5Gew%igen wässrigen Lösung aus Ethylendiamintetraacetat-Natriumsalz (Trilon® B liquid ex BASF) zu der Monomerlösung gegeben.

### Beispiel 2c (Ansatz mit EDTA-Tetranatriumsalz = Trilon® B liquid ex BASF als Prozesshilfsmittel) - nicht erfindungsgemäß

Als Chelatbildner wurden 5,11 g einer 42,5Gew%igen wässrigen Lösung aus Ethylendiamintetraacetat-Natriumsalz (Trilon® B liquid ex BASF) zu der Monomerlösung gegeben.

### Beispiel 3a (Ansatz mit MGDA-Trinatriumsalz = Trilon® M liquid ex BASF als Prozesshilfsmittel) - nicht erfindungsgemäß

Als Chelatbildner wurden 0,65 g einer 40,0Gew%igen wässrigen Lösung aus Methylglycindiacetat-Natriumsalz (Trilon® M liquid ex BASF) zu der Monomerlösung gegeben.

### Beispiel 3b (Ansatz mit MGDA-Trinatriumsalz = Trilon® M liquid ex BASF als Prozesshilfsmittel) - nicht erfindungsgemäß

Als Chelatbildner wurden 6,48 g einer 40,0Gew%igen wässrigen Lösung aus Methylglycindiacetat-Natriumsalz (Trilon® M liquid ex BASF) zu der Monomerlösung gegeben.

### Beispiel 4a (Ansatz mit GLDA-Trinatriumsalz = Dissolvine® GL-47-S ex AkzoNobel als Prozesshilfsmittel) - nicht erfindungsgemäß

Als Chelatbildner wurden 0,71 g einer 47,0Gew%igen wässrigen Lösung aus N,N-bis(carboxymethyl)-L-glutamat-Tetranatriumsalz (Dissolvine® GL-47-S ex AkzoNobel) zu der Monomerlösung gegeben.

### Beispiel 4b (Ansatz mit GLDA-Trinatriumsalz = Dissolvine® GL-47-S ex AkzoNobel als Prozesshilfsmittel) - nicht erfindungsgemäß

Als Chelatbildner wurden 2,86 g einer 47,0Gew%igen wässrigen Lösung aus N,N-bis(carboxymethyl)-L-glutamat-Tetranatriumsalz (Dissolvine® GL-47-S ex AkzoNobel) zu der Monomerlösung gegeben.

### Beispiel 4c (Ansatz mit GLDA-Trinatriumsalz = Dissolvine® GL-47-S ex AkzoNobel als Prozesshilfsmittel) - nicht erfindungsgemäß

Als Chelatbildner wurden 10,21 g einer 47,0Gew%igen wässrigen Lösung aus N,N-bis(carboxymethyl)-L-glutamat-Tetranatriumsalz (Dissolvine® GL-47-S ex AkzoNobel) zu der Monomerlösung gegeben.

### Beispiel 5a (Ansatz mit EDDS-Tetranatriumsalz = Enviomet® C140 ex Innospec Specialty Chemicals als Prozesshilfsmittel) - erfindungsgemäß

Als Chelatbildner wurden 1,37 g einer 35,0Gew%igen wässrigen Lösung aus Ethylendiamindibernsteinsäure-Trinatriumsalz (Enviomet® C140 ex Innospec Specialty Chemicals) zu der Monomerlösung gegeben.

### Beispiel 5b (Ansatz mit EDDS-Tetranatriumsalz = Enviomet® C140 ex Innospec Specialty Chemicals als Prozesshilfsmittel) - erfindungsgemäß

Als Chelatbildner wurden 2,40 g einer 35,0Gew%igen wässrigen Lösung aus Ethylendiamindibernsteinsäure-Trinatriumsalz (Enviomet® C140 ex Innospec Specialty Chemicals) zu der Monomerlösung gegeben.

### Beispiel 5c (Ansatz mit EDDS-Tetranatriumsalz = Enviomet® C140 ex Innospec Specialty Chemicals als Prozesshilfsmittel) - erfindungsgemäß

Als Chelatbildner wurden 5,14 g einer 35,0Gew%igen wässrigen Lösung aus Ethylendiamindibernsteinsäure-Trinatriumsalz (Enviomet® C140 ex Innospec Specialty Chemicals) zu der Monomerlösung gegeben.

### Beispiel 5d (Ansatz mit EDDS-Tetranatriumsalz = Enviomet® C140 ex Innospec Specialty Chemicals als Prozesshilfsmittel) - erfindungsgemäß

Als Chelatbildner wurden 13,71 g einer 35,0Gew%igen wässrigen Lösung aus Ethylendiamindibernsteinsäure-Trinatriumsalz (Enviomet® C140 ex Innospec Specialty Chemicals) zu der Monomerlösung gegeben.

| Beispiel | Maximale Reaktionstemperatur [°C] | Maximaler Hydraulik Differenzdruck an Kneterwelle [bar] | Teabag Retention, WSP241.3 [g/g] | AAP 0.7psi, WSP242.3 [g/g] | Lösliche Anteile 16h, WSP 270.3 [%] vom PrecursorMaterial | Restmonomere, WSP210.3 [mg/kg] vom PrecursorMaterial |
|---|---|---|---|---|---|---|
| Referenz | 82 | 137 | 33,4 | 17,7 | 30,2 | 350 |
| 1* | 78 | 62 | 31,5 | 23,1 | 19,2 | 1710 |
| 2a* | 78 | 96 | 33,9 | 16,0 | 33,0 | 705 |
| 2b* | 77 | 85 | 34,8 | 19,1 | 29,3 | 690 |
| 2c* | 79 | 87 | 33,7 | 17,6 | 31,2 | 655 |
| 3a* | 80 | 99 | 31,9 | 19,2 | 26,1 | 970 |
| 3b* | 78 | 77 | 31,6 | 19,7 | 18,5 | 1510 |
| 4a* | 79 | 89 | 32,6 | 20,6 | 23,0 | 1140 |
| 4b* | 78 | 72 | 32,2 | 21,7 | 20,0 | 1560 |
| 4c* | 78 | 66 | 32,3 | 23,9 | 19,8 | 1340 |
| 5a | 78 | 70 | 32,5 | 22,6 | 21,4 | 1370 |
| 5b | 79 | 62 | 32,9 | 24,2 | 20,7 | 1410 |
| 5c | 82 | 61 | 32,1 | 24,2 | 19,8 | 1660 |
| 5d | 86 | 60 | 31,3 | 25,5 | 15,8 | 2430 |

| | | | | | | |
|---|---|---|---|---|---|---|
| ( )* : nicht erfindungsgemäß | | | | | | |

## Patentansprüche

1. Verfahren zur Herstellung wasserabsorbierender Polymerpartikel durch Polymerisation einer Monomerlösung oder -suspension, die
a) mindestens ein ethylenisch ungesättigtes, säuregruppentragendes Monomer, das zumindest teilweise als Salz vorliegt,
b) mindestens einen Vernetzer,
c) mindestens einen Initiator,
d) wahlweise ein oder mehrere mit den unter a) genannten Monomeren copolymerisierbare ethylenisch ungesättigte Monomere,
e) wahlweise ein oder mehrere wasserlösliche Polymere
f) Wasser
g) wahlweise Additive und/oder Wirksubstanzen
enthält,
wobei das Verfahren weiterhin Trocknung des erhaltenen Polymerisats sowie wahlweise Mahlung des getrockneten Polymerisats und Siebung des gemahlenen Polymerisats sowie wahlweise Oberflächennachvernetzung des getrockneten und gegebenenfalls gemahlenen und gesiebten Polymerisats umfasst, sowie weiterhin wahlweise die Nachbehandlung des ggf. oberflächennachvernetzten Polymerisats mit zumindest einem Nachbehandlungsmittel umfasst, **dadurch gekennzeichnet, dass** die Polymerisation in Gegenwart eines Chelatbildners aus Ethylendiamindibernsteinsäure und/oder ein Salz davon erfolgt und die Polymerisation in einem Kneter durchgeführt wird,
wobei die Zugabe des Chelatbildners Ethylendiamindibernsteinsäure und/oder deren Salz, zu der Monomerlösung oder -suspension vor der Polymerisation erfolgt und/oder wobei die Zugabe des Chelatbildners Ethylendiamindibernsteinsäure und/oder deren Salz, während der Polymerisation zu dem Inhalt des Kneters erfolgt,
wobei der Kneter mit wenigstens zwei parallelen Wellen ausgestattet ist und Elemente an wenigstens einer Welle aufweist, die den Inhalt des Kneters parallel zu den Wellen von einem Zufuhrabschnitt zu einem Ausstoßabschnitt transportieren,
und wobei die resultierenden wasserabsorbierenden Polymerpartikel eine Absorption unter einem Druck von 49,2 g/cm² (0,7 psi) von mindestens 22 g/g aufweisen, gemäß der Edana-Methode Nr. WSP242.3-10 bestimmt.

2. Verfahren nach Anspruch 1, wobei die Monomerlösung weiterhin
d) ein oder mehrere mit den unter a) genannten Monomeren copolymerisierbare ethylenisch ungesättigte Monomere,
e) ein oder mehrere wasserlösliche Polymere und/oder
g) Additive und/oder Wirksubstanzen
enthält.

3. Verfahren nach einem der Ansprüche 1 bis 2, **dadurch gekennzeichnet, dass** der ChelatbildnerEthylendiamindibernsteinsäure und/oder deren Salz, in fester, partikulärer Form oder in Form einer wässrigen Lösung der Monomerlösung oder -suspension zugesetzt wird.

4. Verfahren nach einem der Ansprüche 1 bis 3, **dadurch gekennzeichnet, dass** der Chelatbildner Ethylendiamindibernsteinsäure und/oder deren Salz, in einer Gesamtmenge von zumindest 5 ppm, vorzugsweise 50 bis 2000 ppm, bezogen auf die Menge an unneutralisiertem Monomer der Monomerlösung oder -suspension zugesetzt wird.

5. Verfahren nach einem der Ansprüche 1 bis 4, **dadurch gekennzeichnet, dass** das Monomer a) Acrylsäure ist, die zumindest teilweise als Natriumacrylat vorliegt.

6. Verfahren nach einem der Ansprüche 1 bis 5, **dadurch gekennzeichnet, dass** die Monomerlösung oder -suspension mindestens 0,1 Gew.-% Vernetzer b), bezogen auf unneutralisiertes Monomer a) enthält.

7. Verfahren nach einem der Ansprüche 1 bis 6, **dadurch gekennzeichnet, dass** man eine Oberflächennachvernetzung durchführt.

8. Verfahren nach einem der Ansprüche 1 bis 7, **dadurch gekennzeichnet, dass** man das vorzugsweise oberflächennachvernetze Polymerisat einer Oberflächenbehandlung durch Zugabe zumindest eines Nachbehandlungsmittels unterzieht.

9. Verfahren nach Anspruch 1, **dadurch gekennzeichnet, dass** der Kneter im Batch-Betrieb benutzt wird.

10. Verfahren nach einem der Ansprüche 1 bis 9, **dadurch gekennzeichnet, dass** der Gehalt der Monomerlösung oder -suspension an Eisen-Ionen unter 5 ppm liegt.

11. Verfahren nach einem der Ansprüche 1 bis 10, wobei das Verfahren weiterhin Mahlung des getrockneten Polymerisats und Siebung des gemahlenen Polymerisats umfasst.

## Claims

1. Process for producing water-absorbing polymeric particles by polymerizing a monomer solution or suspension comprising
a) at least one ethylenically unsaturated acid-functional monomer which is present at least partly in salt form,
b) at least one crosslinker,
c) at least one initiator,
d) optionally one or more ethylenically unsaturated monomers copolymerizable with the monomers mentioned under a),
e) optionally one or more water-soluble polymers,
f) water,
g) optionally additives and/or active substances,
wherein said process further comprises drying the polymer obtained and also optionally grinding the dried polymer and sieving the ground polymer and also optionally surface-postcrosslinking the dried and possibly ground and sieved polymer, and also further optionally aftertreating the possibly surface-postcrosslinked polymer with at least one aftertreating agent, **characterized in that** the polymerization is carried out in the presence of a chelating agent from ethylenediaminedisuccinic acid and/or a salt thereof and the polymerization is carried out in a kneader,
wherein the addition of the chelating agent ethylenediaminedisuccinic acid and/or its salt is to the monomer solution or suspension before the polymerization and/or the addition of the chelating agent ethylenediaminedisuccinic acid and/or its salt is to the contents of the kneader during the polymerization, wherein the kneader is equipped with at least two parallel shafts and has elements on at least one shaft to transport the contents of the kneader in parallel with the shafts, from a feed section to an output section, and wherein the resulting water-absorbing polymeric particles have an absorption of at least 22 g/g at a pressure of 49.2 g/cm² (0.7 psi), determined in accordance with Edana method no. WSP242.3-10.

2. Process according to Claim 1 , wherein the monomer solution further comprises
d) one or more ethylenically unsaturated monomers copolymerizable with the monomers mentioned under a),
e) one or more water-soluble polymers and/or
g) additives and/or active substances.

3. Process according to any of Claims 1 to 2, **characterized in that** the chelating agent ethylenediaminedisuccinic acid and/or its salt is added to the monomer solution or suspension in solid, particulate form or in the form of an aqueous solution.

4. Process according to any of Claims 1 to 3, **characterized in that** the chelating agent ethylenediaminedisuccinic acid and/or its salt is added to the monomer solution or suspension in an overall amount of at least 5 ppm, preferably from 50 to 2000 ppm, based on the amount of unneutralized monomer.

5. Process according to any of Claims 1 to 4, **characterized in that** monomer a) is acrylic acid present at least partly in the form of sodium acrylate.

6. Process according to any of Claims 1 to 5, **characterized in that** the monomer solution or suspension comprises at least 0.1% by weight of crosslinker b), based on unneutralized monomer a).

7. Process according to any of Claims 1 to 6, **characterized in that** a surface postcrosslinking is conducted.

8. Process according to any of Claims 1 to 7, **characterized in that** the preferably surface-postcrosslinked polymer is subjected to a surface treatment by addition of at least one aftertreating agent.

9. Process according to Claim 1, **characterized in that** the kneader is used in batch operation.

10. Process according to any of Claims 1 to 9, **characterized in that** the iron ion content of the monomer solution or suspension is below 5 ppm.

11. Process according to any of Claims 1 to 10, wherein the process further comprises grinding the dried polymer and sieving the ground polymer.

## Revendications

1. Procédé de fabrication de particules polymères absorbant l'eau par polymérisation d'une solution ou suspension de monomères, qui contient :
a) au moins un monomère éthyléniquement insaturé, portant des groupes acides, qui se présente au moins en partie sous la forme d'un sel,
b) au moins un agent de réticulation,
c) au moins un initiateur,
d) éventuellement un ou plusieurs monomères éthyléniquement insaturés copolymérisables avec les monomères indiqués en a),
e) éventuellement un ou plusieurs polymères solubles dans l'eau,
f) de l'eau,
g) éventuellement des additifs et/ou des substances actives,
le procédé comprenant en outre le séchage du polymère obtenu, ainsi qu'éventuellement le broyage du polymère séché et le tamisage du polymère broyé, ainsi qu'éventuellement une post-réticulation de surface du polymère séché et éventuellement broyé et tamisé, ainsi qu'en outre éventuellement le post-traitement du polymère éventuellement post-réticulé en surface avec au moins un agent de post-traitement, **caractérisé en ce que** la polymérisation a lieu en présence d'un chélateur constitué par de l'acide éthylène-diamine-disuccinique et/ou un sel de celui-ci, et la polymérisation est réalisée dans un malaxeur,
l'ajout du chélateur acide éthylène-diamine-disuccinique et/ou son sel ayant lieu dans la solution ou suspension de monomères avant la polymérisation, et/ou l'ajout du chélateur acide éthylène-diamine-disuccinique et/ou son sel ayant lieu pendant la polymérisation dans le contenu du malaxeur,
le malaxeur étant équipé d'au moins deux arbres parallèles et comprenant sur au moins un arbre des éléments qui transportent le contenu du malaxeur parallèlement aux arbres depuis une section d'alimentation vers une section de déchargement,
et les particules polymères absorbant l'eau résultantes présentant une absorption sous une pression de 49,2 g/cm² (0,7 psi) d'au moins 22 g/g, déterminée selon la méthode de l'Edana n° WSP242.3-10.

2. Procédé selon la revendication 1, dans lequel la solution de monomères contient en outre :
d) un ou plusieurs monomères éthyléniquement insaturés copolymérisables avec les monomères indiqués en a),
e) un ou plusieurs polymères solubles dans l'eau et/ou
g) des additifs et/ou des substances actives.

3. Procédé selon l'une quelconque des revendications 1 à 2, **caractérisé en ce que** le chélateur acide éthylène-diamine-disuccinique et/ou son sel est ajouté à la solution ou suspension de monomères sous forme particulaire solide ou sous la forme d'une solution aqueuse.

4. Procédé selon l'une quelconque des revendications 1 à 3, **caractérisé en ce que** le chélateur acide éthylène-diamine-disuccinique et/ou son sel est ajouté à la solution ou suspension de monomères en une quantité totale d'au moins 5 ppm, de préférence de 50 à 2 000 ppm, par rapport à la quantité de monomère non neutralisé.

5. Procédé selon l'une quelconque des revendications 1 à 4, **caractérisé en ce que** le monomère a) est l'acide acrylique, qui se présente au moins en partie sous la forme d'acrylate de sodium.

6. Procédé selon l'une quelconque des revendications 1 à 5, **caractérisé en ce que** la solution ou suspension de monomères contient au moins 0,1 % en poids d'agents de réticulation b), par rapport au monomère non neutralisé a) .

7. Procédé selon l'une quelconque des revendications 1 à 6, **caractérisé en ce qu'**une post-réticulation de surface est réalisée.

8. Procédé selon l'une quelconque des revendications 1 à 7, **caractérisé en ce que** le polymère de préférence post-réticulé en surface est soumis à un traitement de surface par ajout d'au moins un agent de post-traitement.

9. Procédé selon la revendication 1, **caractérisé en ce que** le malaxeur est utilisé en mode discontinu.

10. Procédé selon l'une quelconque des revendications 1 à 9, **caractérisé en ce que** la teneur de la solution ou suspension de monomères en ions fer est inférieure à 5 ppm.

11. Procédé selon l'une quelconque des revendications 1 à 10, **caractérisé en ce que** le procédé comprend en outre le broyage du polymère séché et le tamisage du polymère broyé.
